# EUROPEAN PATENT APPLICATION

(11) **EP 1 712 212 A2**
(43) Date of publication of application: **18.10.2006**
(21) Application number: 06114101.6
(22) Date of filing: 27.05.2004
(51) Int. Cl.: A61F 13/15

(54) **Disposable pull-on garment**

(30) Priority: 27.05.2003 US 473582 P
(62) Divisional of application: 04753492.0
(71) Applicant: The Procter and Gamble Company, Cincinnati, Ohio 45202 (US)
(72) Inventor: Matsuda, Toshiyuki, Akashi Hyogo 674-0084 (JP); Fujimoto, Kenji, Kobe Hyogo 658-0013 (JP)
(74) Representative: Heide, Ute

(57) **Abstract**

A disposable pull-on garment is disclosed. The disposable pull-on garment has a waist opening and leg openings. The pull-on garment comprises an absorbent main body, a ring-like elastic belt and an outer cover layer. The absorbent main body comprises a liquid pervious topsheet, a liquid impervious backsheet and an absorbent core disposed therebetween. The absobent main body has a longitudinal centerline, longitudinal side edges, transverse end edges, a front waist region, a back waist region and a crotch region. The front waist region and the back waist region are not joined directly. The ring-like elastic belt comprises a front belt layer and a back belt layer. The ring-like elastic belt is joined to the absorbent main body at the front waist region and the back waist region to form one waist opening and two leg openings. The ring-like elastic belt is not disposed in the crotch region of the absorbent main body. The outer cover layer comprises a separate material from the front belt layer and the back belt layer. The outer cover layer has longitudinal side portions and transverse end portions. The outer cover layer covers the absorbent main body in the crotch region and extends into the front waist region and the back waist region to be joined with the ring-like elastic belt. The longitudinal side portion of the outer cover layer is provided with a leg elastic material to form an elastic leg cuff.

## Description

### CROSS REFERENCE TO RELATED APPLICATION

This application claims the benefit of U.S. Provisional Application No. 60/473,582, filed on May 27, 2003.

### FIELD OF THE INVENTION

The present invention relates to disposable pull-on garments which are donned by inserting the wearer's legs into the leg openings and sliding the garment up into position about the lower torso.

### BACKGROUND OF THE INVENTION

Infants and other incontinent individuals wear disposable absorbent articles such as diapers to receive and contain urine and other body exudates. Absorbent garments having fixed sides (e.g., training pants or pull-on diapers) have become popular for use on children able to walk and often who are toilet training. In order to contain body exudates as well as to fit a wide variety of body shapes and sizes, these pants must fit snugly about the waist and legs of the wearer without drooping, sagging or sliding down from its position on the torso

Many pull-on diapers use elastic elements secured in an elastically contractible condition in the waist and leg openings. Typically, in order to insure full elastic fit about the leg and the waist such as is provided with durable undergarments, the leg openings and waist opening are encircled with elasticized bands of rubber or other materials positioned along the curve of the opening. Examples of such pull-on diapers are disclosed in EP 1 184 012 A1 published on March 6, 2002. The pull-on diaper disclosed therein comprises an absorbent body and an exterior member covering the absorbent body and forming a contour of the diaper. The absorbent body is substantially rectangular and comprises a liquid-permeable topsheet, a liquid-impermeable backsheet and an absorbent core interposed therebetween. The leg and waist elastic members are installed into the exterior member to form an elasticized leg opening and an elasticized waist opening. The leg and waist elastic members should be securely held in a right position of the diaper so that they provide an expected elasticity function around the leg and waist openings. Therefore, the exterior member is formed with two layers comprising an inner layer and an outer layer to hold the leg and waist elastic members therebetween so that the two layers sandwich the leg and waist elastic materials. Although such a pull-on diaper may allow to hold the elastic members in a right position of the diaper, the diaper is bulky and costly because the exterior member is formed with two layers. Especially such a diaper which is bulky in the crotch region is problematic because the bulky material of the crotch region inhibits movement/contraction of leg elastic member and deteriorates fitment about the leg opening and also gives the wearer a stiffish impression and uncomfortableness when in use.

Another example of pull-on diapers is disclosed in Japanese Laid-open Publication No. H4-144558 published on May 19, 1992. The pull-on diaper disclosed therein comprises an absorbent main body and an elastic belt joint to the front region and the back region of the absorbent main body. The elastic belt provides elasticity to hold the diaper on the wearer. The elastic belt is provided to only cover the wearer's waist portion but does not extend into the crotch region of the diaper. This structure lacks an exterior member covering the crotch region of the absorbent main body. Due to the lack of the exterior member, the leg elastic member must be installed into the longitudinal edge of the main absorbent body such that the leg elastic member is interposed between the topsheet and the backsheet. However, such a structure easily causes a leakage problem because the topsheet which typically allows body fluid to wick therethrough extends to the longitudinal edge of the main body.

Based on the foregoing, there is a need for a disposable pull-on garment having a less bulky crotch portion while reducing a leakage problem through the crotch opening. None of the existing disposable pull-on garment provides all of the advantages and benefits of the present invention.

### SUMMARY OF THE INVENTION

The present invention is directed to a disposable pull-on garment. The disposable pull-on garment has a waist opening and leg openings. The pull-on garment comprises an absorbent main body, a ring-like elastic belt and an outer cover layer. The absorbent main body comprises a liquid pervious topsheet, a liquid impervious backsheet and an absorbent core disposed therebetween. The absobent main body has a longitudinal centerline, longitudinal side edges, transverse end edges, a front waist region, a back waist region and a crotch region. The front waist region and the back waist region are not joined directly. The ring-like elastic belt comprises a front belt layer and a back belt layer. The ring-like elastic belt is joined to the absorbent main body at the front waist region and the back waist region to form one waist opening and two leg openings. The ring-like elastic belt is not disposed in the crotch region of the absorbent main body. The outer cover layer comprises a separate material from the front belt layer and the back belt layer. The outer cover layer has longitudinal side portions and transverse end portions. The outer cover layer covers the absorbent main body in the crotch region and extends into the front waist region and the back waist region to be joined with the ring-like elastic belt. The longitudinal side portion of the outer cover layer is provided with a leg elastic material to form an elastic leg cuff.

### BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings and which like designations are used to designate substantially identical elements, and in which:
Figure 1 is a perspective view of the disposable pull-on garment of the present invention in a typical in-use configuration;
Figure 2 is a top plan view of the pull-on garment in its flat uncontracted condition showing the inner surface;
Figure 3 is a cross-sectional view of Figure 2 taken along the line III-III;
Figure 4 is a cross-sectional view of Figure 2 taken along the line IV-IV;
Figure 5 is a cross-sectional view of Figure 2 taken along the line V-V;
Figure 6 is a schematic perspective view of the outer cover layer in a typical in-use configuration without showing other elements of the pull-on garment;
Figure 7 is a schematic top plan view of the outer cover layer of the conventional pull-on garment without showing an absorbent main body;
Figure 8 is a schematic cross-sectional view of Figure 7 taken along the longitudinal centerline L;
Figure 9 is a schematic top plan view of the combination of the ring-like elastic belt and the outer cover layer without showing an absorbent man body;
Figure 10 is a schematic cross-sectional view of Figure 9 taken along the longitudinal centerline L;
Figure 11 is a cross-sectional view showing a typical in-use configuration of the portion shown in Figure 3;
Figure 12 is a top plan view of the pull-on garment in its flat uncontracted condition showing the outer surface;
Figure 13 is a schematic view showing the process for forming the pull-on garment shown in Figure 1; and
Figure 14 is an alternative embodiment of the belt forming section in the process for forming the pull-on garment.

### DETAILED DESCRIPTION OF THE INVENTION

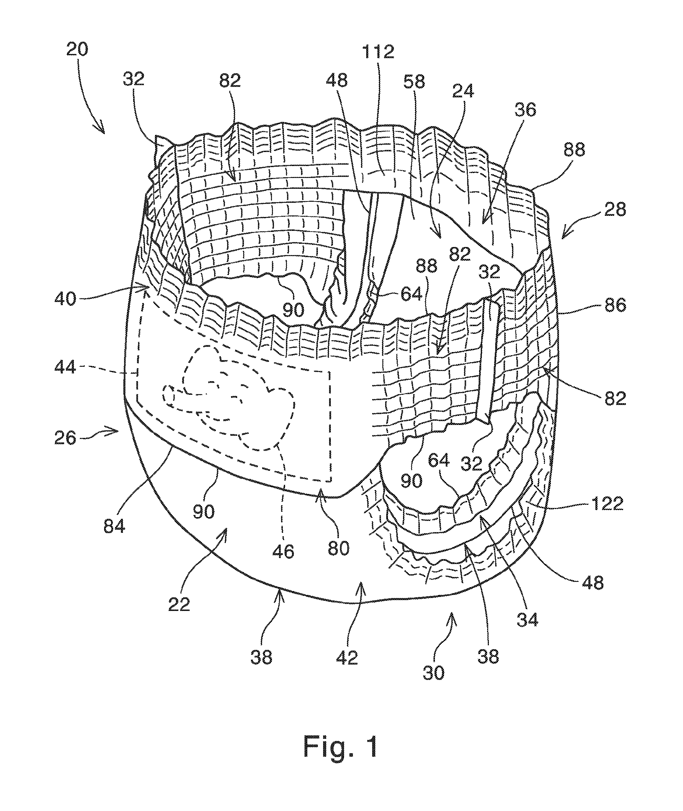

As used herein, the term "pull-on garment" refers to articles of wear which have a defmed waist opening and a pair of leg openings and which are pulled onto the body of the wearer by inserting the legs into the leg openings and pulling the article up over the waist. The term "disposable" is used herein to describe garments which are not intended to be laundered or otherwise restored or reused as a garment (i.e., they are intended to be discarded after a single use and, preferably, to be recycled, composted or otherwise disposed of in an environmentally compatible manner). The pull-on garment is also preferably "absorbent" to absorb and contain the various exudates discharged from the body. A preferred embodiment of the pull-on garment of the present invention is the disposable absorbent pull-on garment, pull-on diaper 20, shown in Figure 1. As used herein, the term "pull-on diaper" refers to pull-on garments generally worn by infants and other incontinent individuals to absorb and contain urine and feces. It should be understood, however, that the present invention is also applicable to other pull-on garments such as training pants, incontinent briefs, feminine hygiene garments or panties, and the like.

Figure 1 is a perspective view of the pull-on diaper 20 of the present invention. The pull-on diaper 20 has an outer surface 22, an inner surface 24 opposed to the outer surface 22, a front region 26, a back region 28, a crotch region 30, and seams 32 which join the front region 26 and the back region 28 to form leg openings 34 and a waist opening 36. The diaper 20 comprises an absorbent main body 38 (hereinafter may be referred to as "main body") to cover the crotch region of the wearer, a ring-like elastic belt 40 (hereinafter may be referred to as "elastic belt" or "belt") extending transversely about the waist opening 36, and an outer cover layer 42 to cover the main body 38. The elastic belt 40 defmes the waist opening 36, and the elastic belt 40 and the main body 38 jointly defmes the leg opening 34. The pull-on diaper 20 also has a patch sheet 44 printed with a graphic 46 thereon which may be disposed in the front region 26 and/or the back region 28.

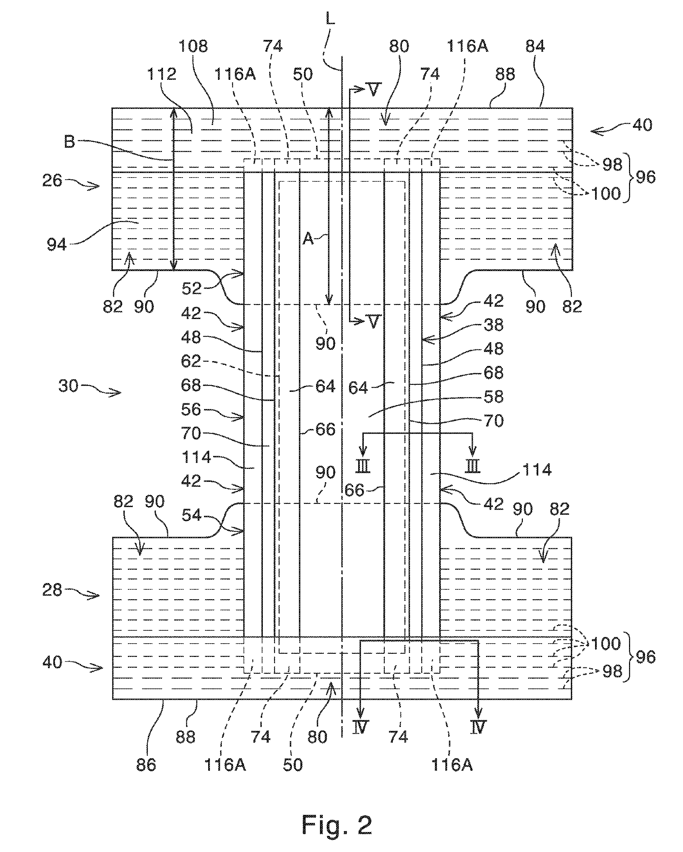

The absorbent main body 38 absorbs and contains body exudates disposed on the main body 38. In the embodiment shown in Figure 2, the main body 38 has a generally rectangular shape having a longitudinal centerline L, longitudinal side edges 48 and transverse end edges 50. The main body 38 also has a front waist panel 52 positioned in the front waist region 26 of the diaper 20, a back waist panel 54 positioned in the back waist region 28, and a crotch panel 56 in the crotch region 30.

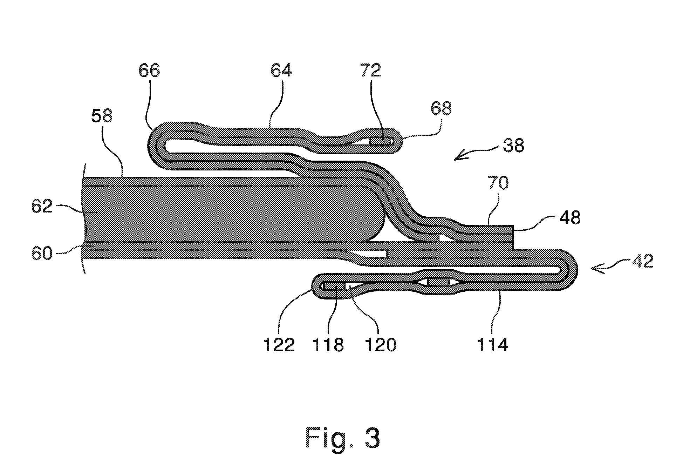

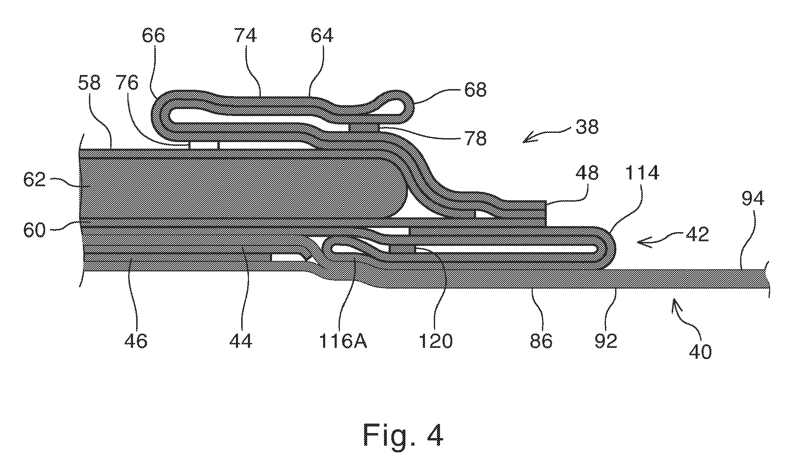

The main body 38 comprises a liquid pervious topsheet 58, a liquid impervious backsheet 60 and an absorbent core 62 disposed therebetween. The main body 38 may additionally comprise a barrier leg cuff 64 disposed along the longitudinal side edge 48. The barrier leg cuff 64 provides improved containment of liquids and other body exudates in the crotch region 30. The barrier leg cuff 64 shown in Figure 3 comprises a single layer of material which is folded into two layers. The barrier leg cuff 64 extends from the longitudinal side edge 48 toward the longitudinal centerline L and then is folded along the folding line 66 back toward the longitudinal side edge 48. The barrier leg cuff 64 has an barrier cuff elastic material 72 at the distal edge 68. The proximal edge 70 of the barrier leg cuff 64 is joined to the backsheet 60 adjacent the longitudinal side edge 48. The portion of the barrier leg cuff 64 along the folding line 66 and the distal edge 68 are free from attachment to any portion of the main body 38 in the crotch panel 56 such that the barrier leg cuff 64 stands up toward the wearer's body when the diaper 20 is used. The transverse end 74 of the barrier leg cuff 64 is joined to the topsheet 58 adjacent the folding line 66 by an attachment means 76 such as an adhesive and is joined to the barrier leg cuff 64 itself along the distal edge 68 by an attachment means 78 as shown in Figure 4.

The liquid pervious topsheet 58 is preferably positioned adjacent the body-facing surface of the absorbent core 62 and may be joined thereto and/or to the backsheet 60 by any attachment means known in the art. The topsheet 58 is preferably compliant, soft feeling, and non-irritating to the wearer's skin. Further, at least a portion of the topsheet 58 is liquid pervious, permitting liquid to readily penetrate through its thickness. A suitable topsheet 58 may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. Preferably, the topsheet 58 is made of a hydrophobic material or is treated to be hydrophobic in order to isolate the wearer's skin from liquids contained in the absorbent core 62. If the topsheet 58 is made of a hydrophobic material, preferably at least the upper surface of the topsheet 58 is treated to be hydrophilic so that liquids will transfer through the topsheet more rapidly.

The liquid impervious backsheet 60 is generally that portion of the diaper 20 positioned adjacent the garment-facing surface of the absorbent core 62. The backsheet 60 prevents the exudates absorbed and contained therein from soiling articles that may contact the diaper 20, such as bedsheets and undergarments. In preferred embodiments, the backsheet 60 is impervious to liquids (e.g., urine) and comprises a thin plastic film. Suitable backsheet materials may include breathable materials that permit vapors to escape from the diaper 20 while still preventing exudates from passing through the backsheet 60.

The absorbent core 62 may comprise any absorbent material that is generally compressible, conformable, non-irritating to the wearer's skin, and capable of absorbing and retaining liquids such as urine and other certain body exudates. The absorbent core 62 can be manufactured in a wide variety of sizes and shapes (e.g., rectangular, hourglass, "T"-shaped, asymmetric, etc.) and may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials. The configuration and construction of the absorbent core 62 may also be varied (e.g., the absorbent core(s) or other absorbent structure(s) may have varying caliper zones, a hydrophilic gradient, a superabsorbent gradient, or lower average density and lower average basis weight acquisition zones; or may comprise one or more layers or structures).

The ring-like elastic belt 40 extends transversely about the waist opening 36 of the diaper 20 and acts to dynamically create fitment forces and to distribute the forces dynamically generated during wear. The elastic belt 40 comprises a front belt 84 and a back belt 86 (hereinafter may be referred to as "front and back belt 84, 86) which are joined at the seam 32 to form a pull-on diaper having a waist opening and two leg openings. Each of the front belt 84 and the back belt 86 has a central panel 80 and side panels 82 positioned transversely outward from the central panel 80. Each of the front belt 84 and the back belt 86 also has a transverse waist border 88 and a transverse abdomen border 90. The transverse waist border 88 defmes the waist opening 36. The central panel 40 of the front belt 84 may partly or entirely overlap with the front waist panel 52 of the main body 38. The central panel 40 of the back panel 86 may partly or entirely overlap with the back waist panel 54 of the main body 38. However, the central panel 40 of the front and back panel 84, 86 does not extend into the crotch panel 56 of the main body 38 and is not disposed in the crotch panel 56. In the embodiment shown in Figure 2, the central panel 40 of the front belt 84 and the back belt 86 partly overlap with the front waist panel 52 and the back waist panel 54, respectively.

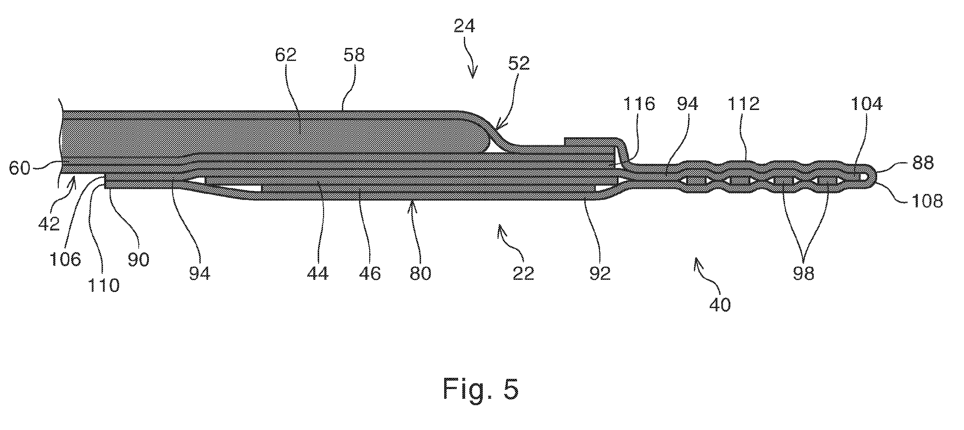

The ring-like elastic belt 40 comprises an outer layer 92. The elastic belt 40 further comprises an inner layer 94 and a belt elastic material 96 interposed between the outer layer 92 and the inner layer 94. The front belt 84 and the back belt 86 may comprise the same materials and/or may have the same structure. Alternatively, the front belt 84 and the back belt 86 may comprise different materials and/or may have different structures. As shown in the embodiment of Figure 2, the front belt 84 and the back belt 86 generally have the same structure. Referring to Figure 5, the inner layer 94 has a transverse waist edge 104 and a transverse abdomen edge 106. The outer layer 92 has a transverse waist end 108 and a transverse abdomen end 110. The inner layer 94 is almost coextensive with the contour of the front and back belt 84, 86. Alternatively, the inner layer 94 may be smaller than the size of the front and back belt 84, 86. The outer layer 92 is longer than the size of the inner layer 94 in the longitudinal direction and an end flap 112 of the outer layer 92 is folded to cover the transverse waist edge 104 of the inner layer 94 at the waist opening 36 and to form a transverse waist end 108 of the outer layer 92. The inner layer 94 may also have an end flap which may be folded together with the end flap 112 of the outer layer 92. The end flap of the inner layer 94 may be longer or shorter than or equal to the end flap of the outer layer 92. Alternatively, the end flap 112 may be eliminated such that the outer layer terminates at the waist opening 36 to form a transverse waist end 108. In the embodiment shown in Figures 2 and 5, the transverse waist end 108 and the transverse abdomen end 110 of the outer layer 92 correspond to the transverse waist border 88 and the transverse abdomen border 90 of the front and back belt 84, 86, respectively. The outer layer 94 surrounded by the transverse waist end 108 and the transverse abdomen end 110 defmes the shape of the front and back belt 84, 86 in the embodiment shown in Figures 2 and 5.

The front and back belt 84, 86 may have any shape to provide a ring-like belt when the front belt 84 and the back belt 86 are joined at the seam 32. In the embodiment shown in Figure 2, the transverse abdomen border 90 may be shaped such that the longitudinal length A between the transverse waist border 88 and the transverse abdomen border 90 in the central panel 80 is longer than the longitudinal length B between the transverse waist border 88 and the transverse abdomen border 90 in the side panel 82. Alternatively, the transverse abdomen border 90 may be straight such that the longitudinal length A and B may be the same in the central panel and in the side panel. Further, the transverse abdomen border 90 may be shaped such that the longitudinal length A may be shorter than the longitudinal length B.

The front and back belt 84, 86 may comprise any known materials. Suitable material for the front and back belt 84, 86 can be manufactured from a wide range of materials such as plastic films; apertured plastic films; woven or nonwoven webs of natural materials (e.g., wood or cotton fibers), synthetic fibers (e.g., polyolefms, polyamides, polyester, polyethylene, or polypropylene fibers), or a combination of natural and/or synthetic fibers; or coated woven or nonwoven webs. Preferably the belt comprises a nonwoven web of synthetic fibers. The belt may comprise a stretchable nonwoven. More preferably, the belt comprises an inner hydrophobic, non-stretchable nonwoven material and an outer hydrophobic, non-stretchable nonwoven material.

The belt elastic material 96 comprises a waist elastic material 98 and a side elastic material 100. The waist elastic material 98 may comprise one or more of elastic elements such as strands or panels extending in the transverse direction. The side elastic material 100 also may comprise one or more of elastic elements such as strands or panels extending in the transverse direction. The waist elastic material 98 is continuously disposed along the transverse waist border 88 of the front and back belt 84, 86. The side elastic material 100 is preferably disposed in the side panel 82 of the front and back belt 84, 86. In the embodiment shown in Figure 2, the waist elastic material 98 and the side elastic material 100 comprise a plurality of elastic strands which are disposed at a constant interval in the longitudinal direction. Alternatively, the waist elastic material 98 and the side elastic material 100 may be disposed at a different interval in the longitudinal direction. No elastic material may be provided in a portion of the central panel 80 of the front and back belt 84, 86 which overlaps with the absorbent core 62, preferably with the front and back waist panel 52, 54 of the main body 38. Alternatively, no elastic material may be provided in the entirety of the central panel 80. However, an elastic material may be provided in the central panel 80 if it is necessary. The belt elastic material 96 is interposed between the outer layer 92 and the inner layer 94 and joined therebetween in a stretched condition of the belt elastic material 96 such that the front and back belt 84, 86 provides elasticity when the diaper 20 is used.

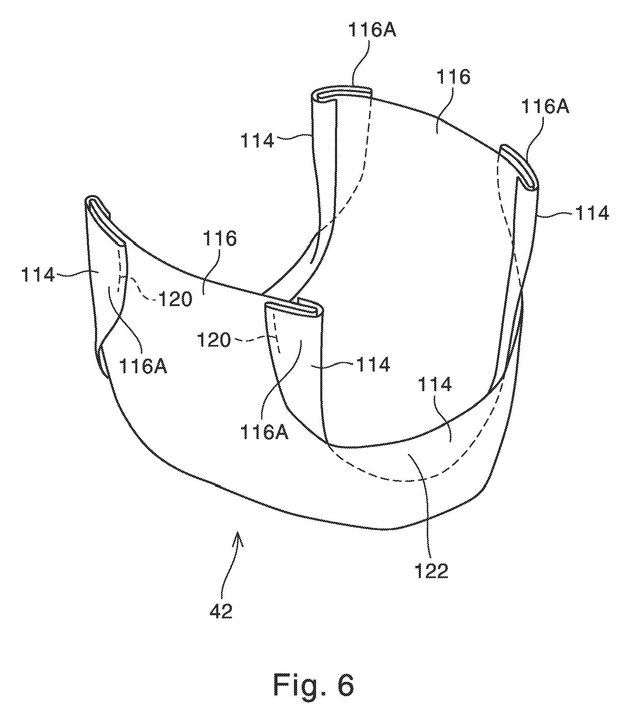

The outer cover layer 42 is disposed on the outer surface 22 of the diaper 20 and covers the crotch panel 56 of the absorbent main body 38. The outer cover layer 42 has a generally rectangular shape having longitudinal side portions 114 and transverse end portions 116 (refer to Figure 5 as well). The longitudinal side portions 114 extends transversely outward beyond the longitudinal side edge 48 of the main body 38. The longitudinal side portion 114 is provided with a leg elastic material 118 to form an elastic leg cuff 122. The leg elastic material 118 is disposed so as to extend generally longitudinally along the longitudinal side portion 114. However, the leg elastic material 118 may be disposed at least in the crotch region 30 of the diaper 20 and may not be disposed along the entirety of the longitudinal side portion 114. In order to form the elastic leg cuff 122 in the embodiment, the longitudinal side portion 114 is folded to form a sleeve 120 to encase the leg elastic material 118 as shown in Figure 3. The transverse end portion 116A along the longitudinal side portion 114 is folded toward the longitudinal centerline L of the main body 38 and tacked down in the folded configuration by any known means such as adhesives, heat bonding, pressure bonding, ultrasonic bonding, etc. The transverse end portion 116A is preferably folded outwardly so as to be away from the liquid impervious backsheet 60 of the main body 38 and is joined onto itself by a bonding means 120 to maintain its folded configuration (refer to Figures 4 and 6). However, the longitudinal side portion 114 in the crotch region 30 is free from attachment (refer to Figures 3 and 6) such that the longitudinal side portion 114 serves as the elastic leg cuff 122 when the diaper 20 is used. Alternatively, the transverse end portion 116A may be folded inwardly onto the topsheet side of the main body 38.

The outer cover layer 42 comprises a material separate from the material of the inner layer 94 and the outer layer 92 constituting the elastic belt 40. Alternatively, the outer cover layer 42 may comprise two of more layer of materials. The outer cover layer 42 may comprise any known materials. Suitable material for the outer cover layer 42 can be manufactured from a wide range of materials such as plastic films; apertured plastic films; woven or nonwoven webs of natural materials (e.g., wood or cotton fibers), synthetic fibers (e.g., polyolefins, polyamides, polyester, polyethylene, or polypropylene fibers), or a combination of natural and/or synthetic fibers; or coated woven or nonwoven webs. Preferably the outer cover layer 42 comprises a single layer of nonwoven web of synthetic fibers. More preferably, the outer cover layer 42 comprises a single layer of hydrophobic, non-stretchable nonwoven material. Further, the outer cover layer 42 may comprise the same material composition as the inner layer 94 and the outer layer 92 of the elastic belt 40.

The outer cover layer 42 covers the crotch panel 56 of the absorbent main body 38. The outer cover layer 42 may extend into and cover the front waist panel 52 and the back waist panel 54 of the main body 38. The outer cover layer 42 is directly joined to and covers the liquid impervious backsheet 42 of the main body 38. In the configuration where the transverse end portion 116A along the longitudinal side portion 114 is folded outwardly so as to be away from the liquid impervious backsheet 60 of the main body and joined onto itself by the bonding means 120, the central panel 80 of the front and back belt 84, 86 is joined to the front waist panel 52 and the back waist panel 54 of the main body 38 through the outer cover layer 42. Thus, the outer cover layer 42 is sandwiched between the front and back belt 84, 86 and liquid impervious backsheet 60 of the main body 38. The transverse end portion 116A tacked down is hidden underneath the front and back belt 84, 86. The front belt 84 and the back belt 86 are joined at the seam 32 to form the ring-like elastic belt 40. However, the front waist panel 52 and the back waist panel 54 are not directly joined along the longitudinal side edges 48 but are only indirectly joined through the front and back belt 84, 86.

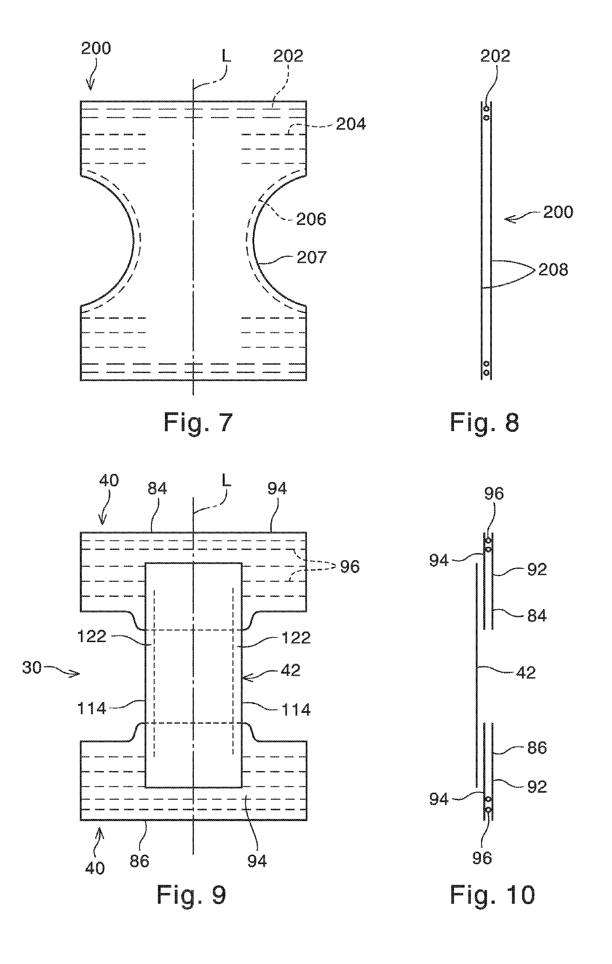

Figure 7 shows a schematic top plan view of an outer cover or an exterior member of a conventional pull-on diaper without showing an absorbent main body. Figure 8 is a schematic cross-sectional view of Figure 7 taken along the longitudinal centerline L. The outer cover 200 is typically coextensive with the contour of the diaper to form a diaper shape and contains an absorbent main body inside. The outer cover 200 has another function which is to hold elastic materials (such as waist elastic material 202, side elastic material 204 and leg elastic material 206) which create fitment forces. In order to hold the elastic materials, the outer cover 200 typically comprises two layers of nonwoven to sandwich and anchor the elastic materials. However, such a structure makes the diaper costly because the outer cover 200 must comprise two layers even in the crotch region. It also makes the crotch region of the diaper makes bulky to inhibit movement/contraction of leg elastic member and deteriorates fitment about the leg opening.

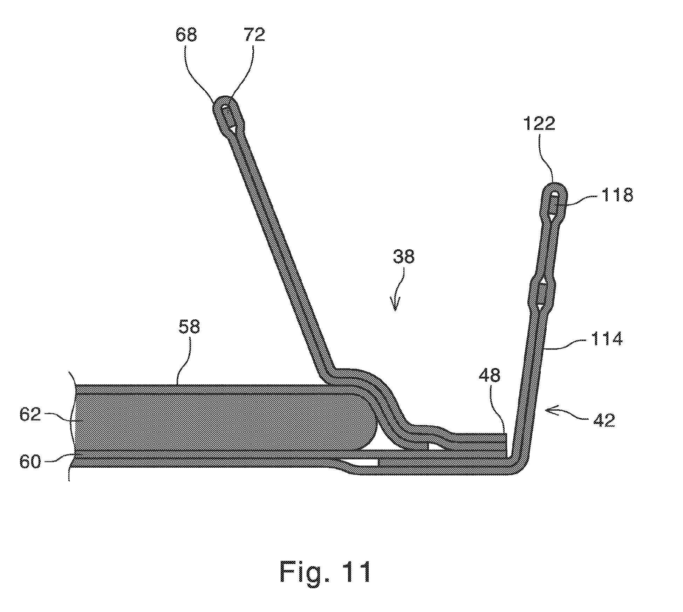

Figure 9 shows a schematic top plan view of a combination of a ring-like elastic belt and an outer cover layer of the present invention without showing an absorbent main body. Figure 10 is a schematic cross-sectional view of Figure 9 taken along the longitudinal centerline L. The front and back belt 84, 86 of the ring-like elastic belt 40 comprises two layers of nonwoven to hold the belt elastic materials 96. However, the ring-like elastic belt 40 formed with two layers of nonwoven (inner layer 94 and outer layer 92) does not extend into the crotch region 30 of the diaper. Instead, the outer cover layer 42 comprising a single layer of nonwoven is disposed in the crotch region 30. This structure is less costly, allows the crotch region 30 of the diaper to be less bulky and eliminates various drawbacks of conventional pull-on diaper. The outer cover layer 42 comprising a nonwoven material also provides a cloth-like appearance together with the ring-like elastic belt 40 comprising a nonwoven material. The outer cover layer 30 is formed with a separate material from the material of the inner layer 94 and the outer layer 92. Therefore, the longitudinal side portion 114 of the outer cover layer 42 can be folded along the longitudinal direction to form the elastic leg cuff 122 or the transverse end portion 116A along the longitudinal side portion 114 can be tacked down as explained above without being interfered by the front and back belt 84, 86. Such folding can be done before the outer cover layer 42 and the front and back belt 84, 86 are joined to one another. The elastic leg cuff 122 formed by the longitudinal side portion 114 extends transversely outward beyond the longitudinal side edge 48 of the main body 38. The elastic leg cuff 122 stands up and covers the longitudinal side edge 48 to reduce leakage from the main body 38 when the diaper 20 is used (refer to Figure 11). Further, the transverse end portion 116A which is outwardly folded and tacked down in its folded configuration ensures the elastic leg cuff 122 to stand up securely in the crotch region 30 (refer to Figure 6 showing a schematic perspective view of the outer cover layer 42 in a typical in-use configuration without showing other elements of diaper 20).

The front and back belt 84, 86 has the shaped transverse abdomen border 90 as explained above. The central panel 80 has the longitudinal length A longer than the longitudinal length B in the side panel 82. This increases the joint area between the central panel 80 and the outer cover layer 42 to enhance the joint strength between the outer cover layer 42 and the central panel 80 of the front and back belt 84, 86. On the other hand, the longitudinal length B in the side panel 82 being shorter than the longitudinal length A allows to form leg openings with a greater circumference for providing comfortableness to the wearer.

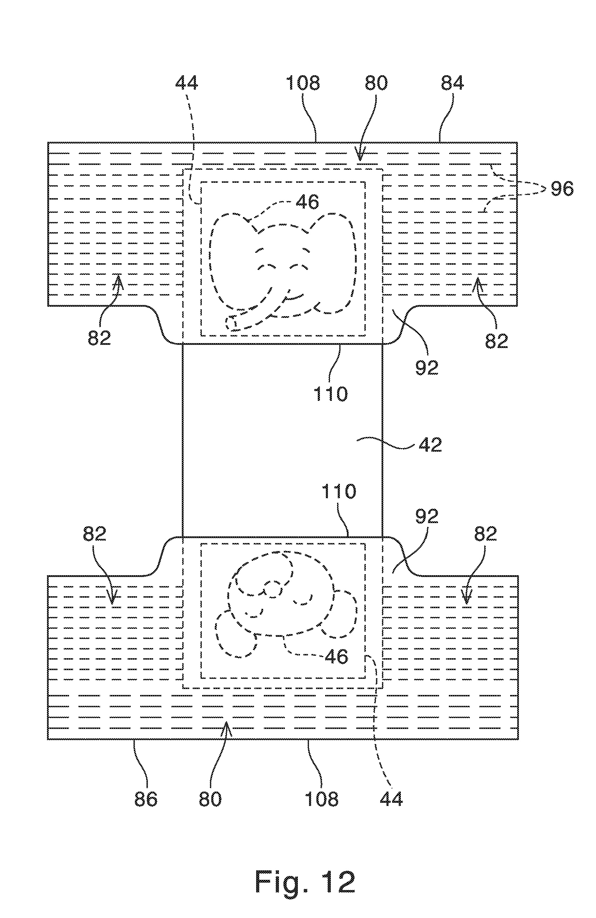

The patch sheet 44 printed with a graphic 46 is provided on the diaper 20 to provide an aesthetic appearance. The graphic 46 may be any graphic to increase aesthetic appearance, such as visual characters, educational signs or marks. The patch sheet 44 may comprise any known material such as a plastic film, a woven, a nonwoven or tissues and may have any shape. The patch sheet 44 may also comprise a single sheet or two or more separate sheets. In the embodiment shown in Figure 12, the patch sheet 44 comprises a single rectangular nonwoven having high breathability. The printing may be made by any known process such as flexographic printing, ink-jet printing, screen printing, or rotogravure printing.

The patch sheet 44 is a material separate from any elements constituting the diaper 20. The patch sheet 44 may be joined anywhere as far as it can be seen by the user of the diaper. The patch sheet 44 is preferably joined somewhere outside the liquid impervious backsheet 60 of the main body 38, preferably outside the outer cover layer 42, more preferably outside the inner layer 94 of the front and back belt 84, 86 to reduce hazy appearance of the graphic 46. The patch sheet 44, however, is joined inside the outer layer 94 of the front and back belt 84, 86 to prevent an ink rub-off problem caused by abrasion of the ink layer of the graphic 46 with other substrates such as cloths or floors. In the embodiment shown in Figure 5, the patch sheet 44 with the graphic 46 is disposed and joined between the inner layer 92 and the outer layer 94 of the front and back belt 84, 86. However, if the front and back belt 84, 86 of the diaper 20 is formed with only the belt elastic material 96 and the outer layer 94 and does not have the inner layer 92, the patch sheet 44 may be disposed between the liquid impervious backsheet 60 of the main body 38 and the outer layer 94 of the front and back belt 84, 86. In such a case, the patch sheet 44 may be joined to the liquid impervious backsheet 60, the outer layer 94 or both of them.

The position of the patch sheet 44 is selected such that the patch sheet 44 is disposed between the transverse waist end 108 and the transverse abdomen end 110 of the front and back belt 84, 86. The transverse waist end 108 and the transverse abdomen end 110 do not cross any portion of the graphic 46 (refer to Figure 12). The entirety of the graphic 46 is covered by only the outer layer 92. Therefore, the graphic appearance is the substantially same anywhere in the patch sheet 44 not to make a part of the graphic 46 to have hazier appearance than other parts of the graphic 46. While the size of the patch sheet 44 may be restricted by the shape of the front and back belt 84, 86, the size of the patch sheet 44 is still adjustable by making the transverse abdomen border 90 (which corresponds to the transverse abdomen end 110 in the embodiment) of the front and back belt 84, 86 shaped. The central panel 80 having the longitudinal length A longer than the longitudinal length B in the side panel 82 (refer to Figure 2) allows to use the patch sheet 44 having a longer longitudinal length than a patch sheet installed in the front and back belt having a straight transverse waist border and a straight abdomen border.

The patch sheet 44 is preferably disposed in the central panel 80 of the front and back panel 84, 86 in which the belt elastic material 96 is not present as shown in Figure 12. However, the patch sheet 44 may be disposed in the area such as in the side panel 82 in which the belt elastic material 96 is present. The patch sheet 44 may be coextensive with the outer layer 92 of the front and back belt 84, 86 such that the patch sheet 44 has the same shape as the outer layer 92. However, it is preferable that the patch sheet 44 is smaller than the outer layer 92 to reduce bulkiness of the front and back belt 84, 86. The front and back belt 84, 86 may have two or more patch sheets with a graphic which are disposed between the transverse waist end 108 and the transverse abdomen end 110 of the front and back belt 84, 86.

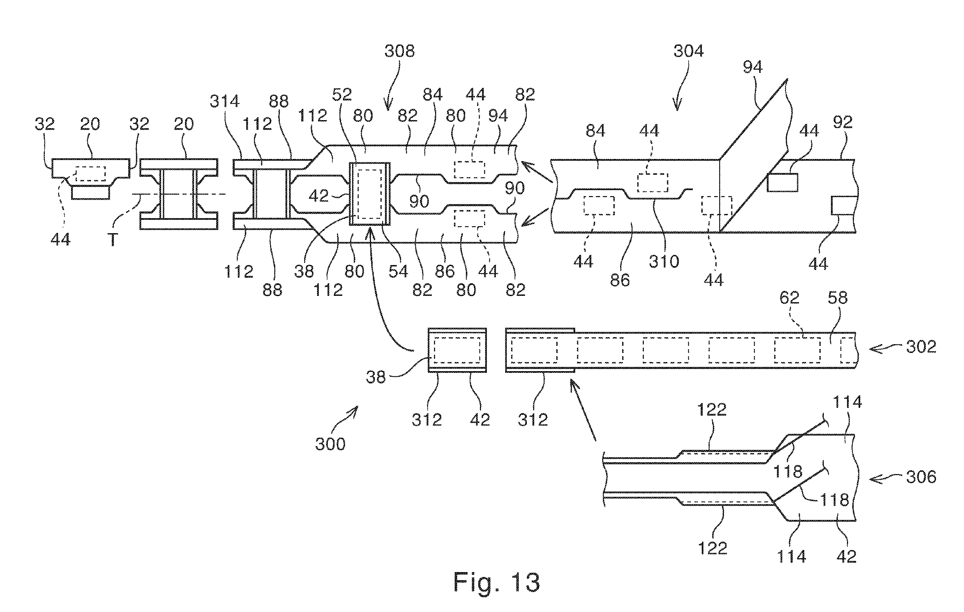

Figure 13 shows a schematic view to explain a process for forming the diaper 20. The process 300 shown in Figure 13 primarily comprises four sections; a main body forming section 302, a belt forming section 304, an outer cover layer forming section 306 and an assembly section 308. The belt elastic material is not illustrated in Figure 13.

The main body forming process 302 combines elements forming the main body 38 such as the topsheet 58, the backsheet 60, the absorbent core 62 and the barrier leg cuff 64 (Figure 13 shows only the topsheet 58 and the absorbent core 62) such that the absorbent core 62 is sandwiched between the topsheet 58 and the absorbent core 62. These elements are joined to each other by any known means such as adhesives.

The belt forming section 304 combines the outer layer 92, the inner layer 94 and the patch sheet 44 and cuts the combined web comprising the outer layer 92 and the inner layer 94 into the front belt 84 and the back belt 86 along the cut line 310 which corresponds to the transverse abdomen border 90 of the front and back belt 84, 86. The patch sheet 44 is disposed in the front and back belt 84, 86 alternately in a zigzag configuration as shown in Figure 13 and arranged so that the patch sheet is positioned in the central panel 80 of the front and back belt 84, 86. The cut line 310 is shaped such that the transverse abdomen border 90 is also shaped. The transverse abdomen border 90 protrudes in the central panel 80 compared with the transverse abdomen border 90 in the side panel 82. The belt elastic material 96 (not shown in Figure 13) is also joined between the inner layer 94 and the outer layer 92. Then the front belt 84 and the back belt 86 are separated from each other and are phased such that the shaped transverse abdomen borders 90 of the front and back belt 84, 86 are symmetric with respect to the direction in which the front and back belt 84, 86 is forwarded.

The outer cover layer forming section 306 forms the outer cover layer 42 having the elastic leg cuff 122. The outer cover layer 122 is provided with the leg elastic material 118 along the longitudinal side portion 114. The longitudinal side portion 114 is folded as shown in Figure 13 to form the elastic leg cuff 122. After that, the longitudinal side portion 114 is oppositely folded and the portion corresponding to the transverse end portion 116A is tacked down in the folded configuration by the bonding means 120 (refer to Figure 4). The outer cover layer 42 thus provided with the elastic leg cuff 122 is joined to the backsheet side of the main body 38 to form the intermediate assembly 312 comprising the main body 38 and the outer cover layer 42. The intermediate assembly 312 is then cut into the individual intermediate assembly 312. The individual intermediate assembly 312 is turned by 90 degree and fed into the assembly section 308.

The assembly section 308 combines the individual intermediate assembly 312 with the front and back belt 84, 86. The individual intermediate assembly 312 is provided on the front and back belt 84, 86 such that the front waist panel 52 and the back waist panel 54 of the main body 38 is joined through the outer cover layer 42 to the central panel 80 of the front and back belt 84, 86, respectively. After that, the end flap 112 of the front and back belt 84, 86 is folded inwardly along the transverse waist border 88 to form a continuous diaper assembly 314 comprising the main body 38, the outer cover layer 42 and the front and back belt 84, 86. The continuous diaper assembly 314 thus formed is cut into each individual diaper 20. The individual diaper 20 is then folded along the transverse centerline T in the crotch region and the side panel 82 of the front and back belt 84, 86 is joined at the seam 32.

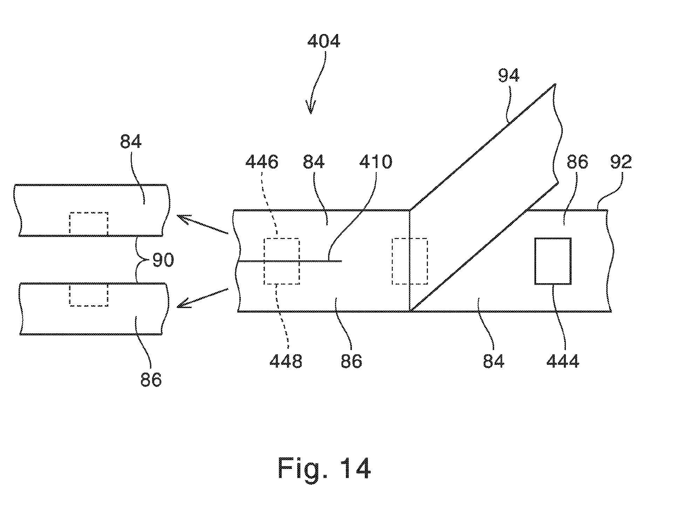

Figure 14 shows an alternative embodiment of the belt forming section. The belt forming section 404 combines the outer layer 92, the inner layer 94 and the patch sheet 444. The patch sheet 444 in Figure 14 is positioned to extend both in the front belt 84 and in the back belt 86. The combined web is then cut into the front and belt 84 and the back belt 86 along the cut line 410 which corresponds to the transverse abdomen border 90 of the front and back belt 84, 86. The patch sheet 444 is also cut into the front patch sheet 446 and the back patch sheet 448. The cut line 410 is straight such that the transverse abdomen border 90 is also straight.

All documents cited in the Detailed Description of the Invention are, are in relevant part, incorporated herein by reference; the citation of any document is not to be construed as an admission that it is prior art with respect to the present invention.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A disposable pull-on garment having a waist opening and leg openings, the pull-on garment comprising an absorbent main body, a ring-like elastic belt and an outer cover layer, wherein
the absorbent main body comprises a liquid pervious topsheet, a liquid impervious backsheet and an absorbent core disposed therebetween, and the absorbent main body has a longitudinal centerline, longitudinal side edges, transverse end edges, a front waist panel, a back waist panel and a crotch panel, the front waist panel and the back waist panel being not joined directly,
the ring-like elastic belt has a central panel and side panels, the central panel is joined to the front waist panel and the back waist panel of the absorbent main body to form one waist opening and two leg openings, the ring-like elastic belt is not disposed in the crotch panel of the absorbent main body,
the outer cover layer comprises a separate material from the ring-like elastic belt, the outer cover layer has longitudinal side portions and transverse end portions, the outer cover layer covers the crotch panel of the absorbent main body and extends into the front waist panel and the back waist panel to be joined with the ring-like elastic belt, and the longitudinal side portion of the outer cover layer is provided with a leg elastic material to form an elastic leg cuff.

2. A disposable pull-on garment of Claim 1 wherein the longitudinal side portion extends transversely beyond the longitudinal side edge of the absorbent main body.

3. A disposable pull-on garment of Claim 2 wherein the longitudinal side portion of the outer cover layer is folded to form a sleeve to encase the leg elastic material.

4. A disposable pull-on garment of Claim 2 or 3 wherein the transverse end portion along the longitudinal side portion of the outer cover layer is folded toward the longitudinal centerline of the main absorbent body and are tacked down in the folded configuration.

5. A disposable pull-on garment of Claim 4 wherein the transverse end portion along the longitudinal side portion of the outer cover layer is folded outwardly so as to be away from the liquid impervious backsheet of the main absorbent body.

6. A disposable pull-on garment of Claim 1 wherein the outer cover layer covers and is joined to the liquid impervious backsheet of the absorbent main body in the crotch panel.

7. A disposable pull-on garment of Claim 6 wherein the outer cover layer is positioned and joined between the liquid impervious backsheet and the ring-like elastic belt in the front waist panel and in the back waist panel.

8. A disposable pull-on garment of Claim 1 wherein the outer cover layer comprises a single layer of hydrophobic nonwoven material.

9. A disposable pull-on garment of Claim 1 or 8 wherein the ring-like elastic belt comprises two layers of hydrophobic nonwoven material and a belt elastic material disposed therebetween.
